# EUROPEAN PATENT APPLICATION

(11) **EP 4 169 531 A1**
(43) Date of publication of application: **26.04.2023**
(21) Application number: 21807721.2
(22) Date of filing: 19.05.2021
(51) Int. Cl.: A61K 45/00, A61P 11/00, A61P 29/00, A61P 31/12, A61P 31/14, A61P 37/02, C07K 2/00, C07K 5/037, C07K 5/093, A61K 38/06, A61K 38/08, C12N 9/99, A61K 31/198

(54) **MEDICAL AGENT CONTAINING ACTIVE SULFUR COMPOUND AS MAIN INGREDIENT**

(30) Priority: 20.05.2020 JP 2020087899; 01.10.2020 JP 2020167343
(71) Applicant: Tohoku University, Sendai-shi, Miyagi 980-8577 (JP); Bio-Xcelerator, Inc., Tokyo 160-0016 (JP)
(72) Inventor: AKAIKE, Takaaki, Sendai-shi, Miyagi 980-8575 (JP); TAKAGI, Satoshi, Tokyo 160-0016 (JP); SUGIURA, Hisatoshi, Sendai-shi, Miyagi 980-8575 (JP); INABA, Kenji, Sendai-shi, Miyagi 980-8577 (JP)
(74) Representative: Mewburn Ellis LLP
(86) International application number: PCT/JP2021/019023
(87) International publication number: WO 2021/235494

(57) **Abstract**

There is provided a drug which exhibits preventive and therapeutic effects on virus infectious diseases by inhibiting virus-derived protease on the basis of various physiological activity actions of an active sulfur compound(s) which exists in living organisms.

## Description

### TECHNICAL FIELD

The present invention relates to a drug with an active sulfur compound as its main medical efficacy component.

### BACKGROUND ART

The physiological importance of reactive sulfur molecular species / active sulfur molecular species (reactive sulfur species, RSS) such as cysteine hydropolysulfide (CysSSH) and glutathione polysulfide (GSSH) is reported (NPL 1).

Cysteine hydropolysulfide (CysSSH) occurs in abundant quantities in various organisms, yet little is known about its biosynthesis and physiological functions. Extensive persulfide formation is apparent in cysteine-containing proteins in Escherichia coli and mammalian cells and is believed to result from post-translational processes involving chemical reactions related to sulfur metabolites in living organisms.

There is effective CysSSH synthesis from the substrate L-cysteine, which is a reaction catalyzed by prokaryotic and mammalian cysteinyl-tRNA synthetases (CARSs). Targeted disruption of the genes encoding mitochondrial CARSs in mice and human cells shows that CARSs serve a crucial role in endogenous CysSSH production and suggests that these enzymes serve as the principal cysteine persulfide synthetases in vivo. CARSs also catalyze co-translational cysteine polysulfidation and are involved in the regulation of mitochondrial biogenesis and bioenergetics.

Therefore, elucidation of the mechanism for producing CARS-dependent persulfide metabolites may possibly help understanding of aberrant redox signaling in physiological and pathophysiological conditions, and suggest therapeutic targets based on oxidative stress and mitochondrial dysfunction.

The in vivo kinetics of a considerable amount of RSS generated endogenously and ubiquitously in both prokaryotes and eucaryotes will become clear by establishing active sulfur metabolomics analysis by using RSS metabolic profiling. However, chemical properties of polysulfides are not completely understood or elucidated due to their reactivity or complicated redox active property.

Meanwhile, there is an urgent need to elucidate clinical conditions of a novel coronavirus (SARS-CoV-2) which causes a novel coronavirus infection (COVID-19) and establish its prevention and treatment method. SARS-CoV-2 protease inhibitors are reported as therapeutic drugs for the COVID-19 (NPL 2 and NPL 3). Incidentally, WO/2017/005768 publication discloses that it has a problem of providing an intraocular perfusate which sufficiently protect intraocular tissues during an eye surgery and is highly safe and the problem is solved by the intraocular perfusate containing at least one type of active sulfur molecules represented by cysteine persulfide and glutathione persulfide.

### CITATION LIST

### PATENT LITERATURE

PTL 1: WO/2017/005768

### NON-PATENT LITERATURE

NPL 1: NATURE COMMUNICATIONS |8: 1177, Cysteinyl-tRNA synthetase governs cysteine polysulfidation and mitochondrial bioenergetics, 27 October 2017.
NPL 2: Zhang L, et al. Crystal structure of SARS-CoV-2 main protease provides a basis for design of improved α ketoamide inhibitors. Science 368:409-412 (2020).
NPL 3: Jin Z, et al. Structure of Mpro from COVID-19 virus and discovery of its inhibitors. Nature 582:289-293 (2020).

### SUMMARY OF THE INVENTION

### PROBLEMS TO BE SOLVED BY THE INVENTION

The present invention has a problem of providing a drug which exhibits preventive and therapeutic effects on virus infectious diseases by inhibiting virus-derived proteases on the basis of various physiological activity actions of active sulfur compounds which exist in living organisms, and it is an object of the present invention to provide the above-described drug. NPL 3 described above neither discloses nor suggests this.

### MEANS TO SOLVE THE PROBLEMS

In order to achieve the above-described object, a first invention is characterized in that it is an antiviral drug containing an active sulfur compound as its main medical efficacy component. The active sulfur compound is, for example, R₁S(S)ₙR₂ (where R₁ and R₂ are selected from amino acids and polypeptides, which independently have a thiol group, and represent parts other than the thiol group; and n is an integer equal to or more than 2 and preferably 7 or less). The active sulfur compound is formed so that, for example, each of R₁ and R₂ is L-cysteine (Cys), homocysteine (Hcys), or glutathione (GSH). The active sulfur compound is, for example, glutathione polysulfide and, particularly, GSSSG.

The virus is a coronavirus (CoV) and, particularly, a novel coronavirus (SARS-CoV-2). The antiviral drug is, for example, a preventive and therapeutic drug for SARS-CoV-2 pneumonia.

A second invention is a preventive and therapeutic drug for the novel coronavirus infection (COVID-19), which contains glutathione poly(tri)sulfide (such as GSSSG) as its main medical efficacy component; a third invention is an immune control agent (adjuster) containing glutathione poly(tri)sulfide as its main medical efficacy component; and a fourth invention is an anti-inflammatory drug containing glutathione poly(tri)sulfide as its main medical efficacy component.

A fifth invention is a pneumonia preventive and therapeutic drug for a viral pneumonia or the like, which contains an active sulfur compound as its main component. Aspects of the active sulfur compound are as described earlier.

### ADVANTAGEOUS EFFECTS OF THE INVENTION

The drug which exhibits preventive and/or therapeutic effects on the virus infectious diseases by, for example, inhibiting the virus-derived protease on the basis of various physiological activity actions of the active sulfur compound(s) existing in vivo can be provided according to the present invention.

### BRIEF DESCRIPTION OF DRAWINGS

[Fig. 1]

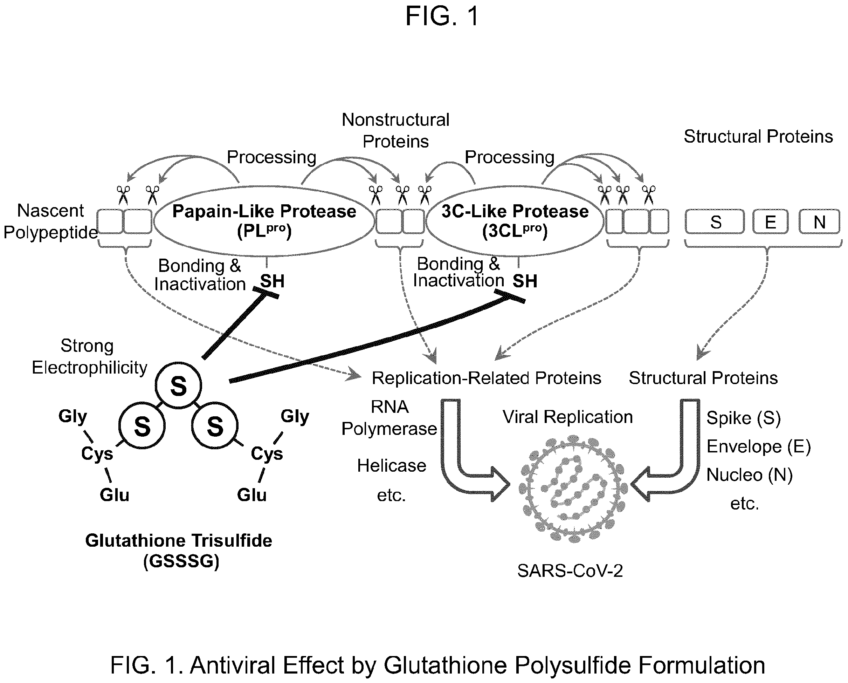

   Fig. 1 is a block diagram illustrating an antiviral effect by glutathione polysulfide formulation.
[Fig. 2]

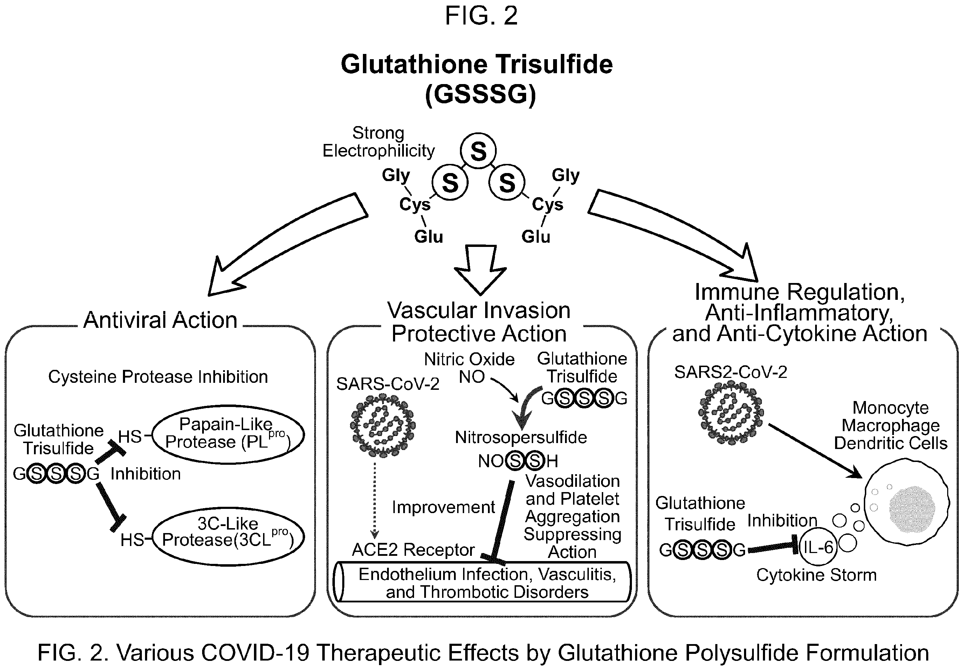

   Fig. 2 is a diagram illustrating various COVID-19 therapeutic effects by glutathione polysulfide formulation.
[Fig. 3]

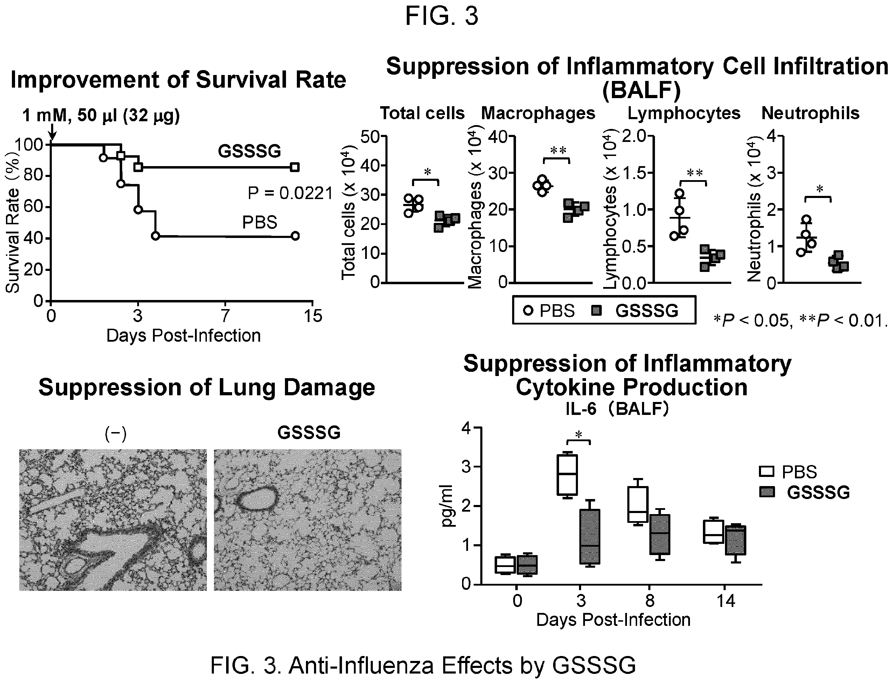

   Fig. 3 is a graph illustrating anti-influenza effects by GSSSG.
[Fig. 4]

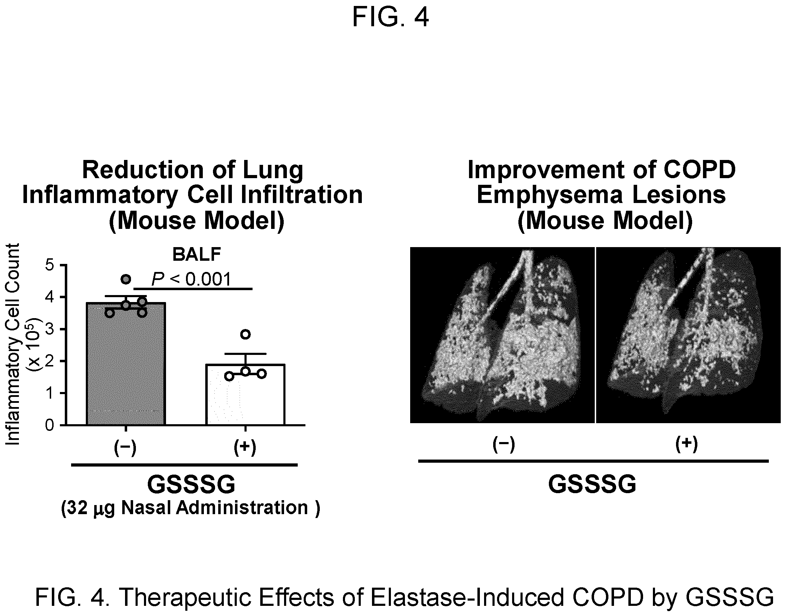

   Fig. 4 is a graph illustrating therapeutic effects on an elastase-induced COPD by the GSSSG.
[Fig. 5]

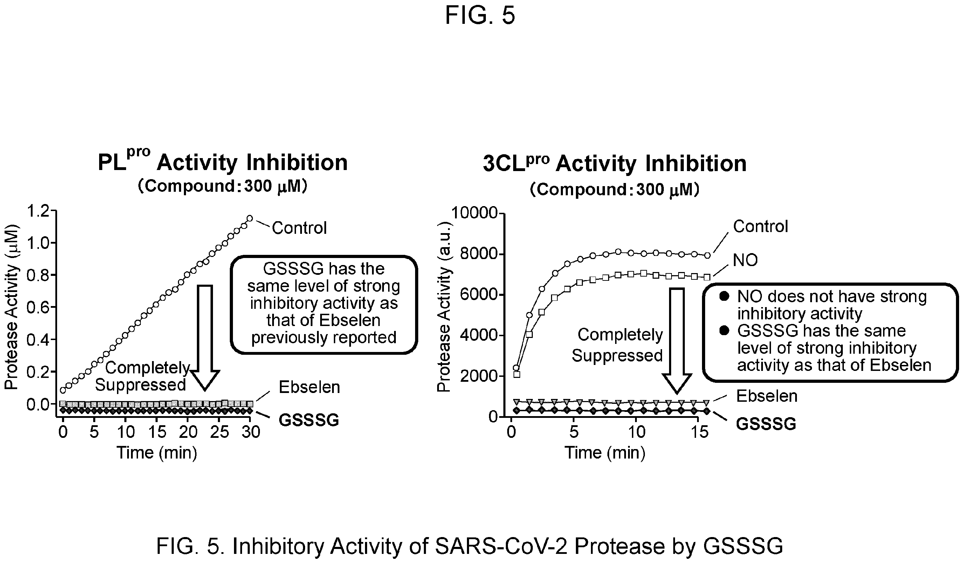

   Fig. 5 is a graph illustrating a SARS-CoV-2 protease inhibitory effect by the GSSSG.
[Fig. 6]

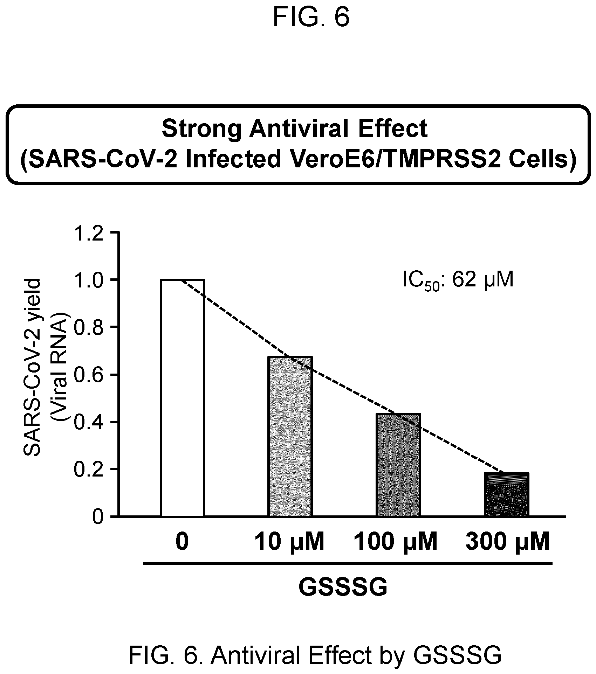

   Fig. 6 is a graph illustrating an antiviral effect by the GSSSG.

### DESCRIPTION OF EMBODIMENTS

The SARS-CoV-2 expresses two different cysteine(thiol) proteases based on its own virus genomes. They are a papain-like protease (PLpro) and a 3C-like protease (3CLpro or also called a main protease, Mpro) and these proteases are essential for replication within host cells of the virus. Crystal structures of these proteases have been clarified.

The inventors established the invention which strongly inhibits enzyme activity of PLpro and 3CLpro by causing particularly glutathione polysulfide (such as glutathione trisulfide, GSSSG (G: glutathione)), which is a donor of active sulfur molecules and is one of the active sulfur compounds, to covalently bond specifically with the thiol group which is the activity center of these two proteases, PL^{pro} and 3CL^{pro}. It has already been reported that as a result, Ebselen which indicates a similar inhibition style exhibits a conspicuous proliferation suppression effect on the novel coronavirus (SARS-CoV-2) (NPL 2 and NPL 3 mentioned earlier). Since the electrophilicity of sulfur (GSSSG) at the center of an oxidized form of the polysulfide is stronger than that of a reduced form of the polysulfide (GSSSH), the oxidized form of polysulfides is generally more useful.

The inventors of the present application: invented a new anti-SARS-CoV-2 drug by using a direct antiviral effect of the GSSSG as glutathione polysulfide caused by inhibiting the protease activity of SARS-CoV-2 and its variants (Fig. 1); and further achieved an invention of an integrative anti-COVID-19 therapeutic agent which is not just a simple anti-virus therapy, by utilizing strong inflammation and immune control activity (inflammation and immune suppression) of the GSSSG (Fig. 2) and thereby preventing the occurrence of intractable diseases such as a cytokine storm which is a clinical condition of severe (fulminant) pneumonia of COVID-19, and interstitial pneumonia and pulmonary fibrosis which are considered as important as complications or aftereffects (complications) of pneumonia of COVID-19 (Fig. 2). The glutathione polysulfide exhibits a suppression effect on the inflammation as a result of the virus infection such as the SARS-CoV-2.

Referring to Fig. 2, the glutathione trisulfide achieves an antiviral action by inhibiting two cysteine proteases. The glutathione trisulfide suppresses production of interleukin-6 by an immune response induced by the SARS-CoV-2 infection and achieves immune control and anti-inflammatory, anti-cytokine actions. Furthermore, the glutathione trisulfide reacts and bonds with nitric oxide (NO) and enhances vasodilation and platelet aggregation suppressing actions by NO, thereby exhibiting a strong vascular protection action with respect to thrombus formation, vasculitis, and vascular injuries which are known as complications of the COVID-19.

Virus protease inhibitors have been actively searched for the development of antiviral agents. However, many compounds specialize only either one of the proteases and, in fact, just have the inhibitory activity limited only to each protease and there is no precedent of a virus protease inhibitor like the glutathione polysulfide (GSSSG) which exhibits strong medical efficacy by inhibiting the two cysteine proteases of the SARS-CoV-2 at the same time.

The GSSSG exhibits a therapeutic effect on the viral pneumonia via its strong anti-inflammatory action. Practically, it has been found that preventive and therapeutic effects on influenza A virus pneumonia are exhibited by nasal administration of the GSSSG to mice (Fig. 3). Fig. 3 shows the results of examples of the anti-influenza effect by the GSSSG. An influenza virus PR8 strain (A/PR/8/34 (H1N1), Akaike T. et al, Journal of Infectious Diseases, 170, 1023-1026, 1994) (2000 pfu/ml (PBS)) was administered to each of a plurality of B6 mice (ten 8-week-old mice for each group). This administration was performed by intratracheal administration of a virus solution (PBS 40 µL) under anesthesia. Furthermore, nasal administration of GSSSG (50 µL,1 mM) was performed under anesthesia six hours before the virus inoculation and then a survival rate of the mice, their lung damage (14 days after infection), and various kinds of inflammatory cell counts (three days after infection) were measured chronologically and analyzed. A group to which the GSSSG was administered was compared with a control group (PBS-administered group / no GSSG administered) concerning the survival rate, whether there is any lung damage or not, and the various kinds of inflammatory cell counts. Regarding the lung damage, pneumonia images were evaluated by means of HE staining. Regarding the measurement of the inflammatory cell counts, bronchoalveolar lavage (BAL) was performed by using PBS and the number of cells in BAL and cytokine were analyzed.

The therapeutic effect of the GSSSG was also confirmed with respect to bronchial asthma, chronic obstructive pulmonary disease (COPD), and pulmonary fibrosis models (Fig. 4). Idiopathic interstitial pneumonia and pulmonary fibrosis are reported as aftereffects of SARS which was experienced in the past, and there are concerns about the occurrence of similar refractory lung disease regarding the COVID-19; however, the GSSSG brings about wide preventive and therapeutic effects on all the clinical conditions through the entire process from the beginning of the above-described SARS-CoV-2 infection to the late stage of infection, convalescence, and complications.

Fig. 4 is an example indicating the therapeutic effect of the GSSSG on elastase-induced COPD; and airway administration of GSSSG (50 µL, 1 mM) to porcine pancreas elastase (PPE) induced emphysema models of CARS2-deficient B6 mice^{*1} was performed three times, that is, six hours before, two days later, and four days later, and an inflammatory cell count in a bronchial alveolar lavage fluid (BALF) five days after the PPE administration was measured and emphysema lesions were analyzed by using CT. The inflammatory cell count was measured in the same manner as in the example of Fig. 3. It has been confirmed that the inflammatory cell count of the GSSSG-administered group (GSSSG(+)) is small as compared to that of the no GSSSG-administered group (GSSSG(-) / control group) and, as you can see from images obtained by CT, remission of the emphysema has been achieved as compared to the no GSSSG-administered group.
*1: NATURE COMMUNICATIONS |8: 1177, Cysteinyl-tRNA synthetase governs cysteine polysulfidation and mitochondrial bioenergetics, 27 October 2017.

Various active sulfur molecules exist in living organisms and an example of main molecules among them is GSSSG which is glutathione with excessive sulfur molecules attached thereto. Since this is relatively stable and can be synthesized in a large amount and with high purity, the inventors have examined its medical efficacy. Meanwhile, it has been found that such active sulfur is also formed in a thiol group of cysteine in proteins; and, for example, it has been clarified that the activity of thiol in cysteine which is the activity center of thiol protease such as papain is suppressed by this sulfur addition reaction (persulfidation).

The inventors have also found that the GSSSG not only exhibits the effect of improving severe pneumonia of the influenza pneumonia models of the mice, but also alleviates respiratory tract and lung damage and pulmonary fibrosis via suppression of production of inflammatory cytokine in bronchial asthma, COPD, and pulmonary fibrosis models of the mice. It is generally known that patients with the COVID-19 who have such underlying health conditions may become severely ill. As cytokine such as IL-6 increases in the aforementioned CARS2-deficient mice, the anti-cytokine activity of the active sulfur species (RSS) such as GSSSG was confirmed.

Regarding the administration of the drug, there is a major advantage of being capable of locally administering the GSSSG as an inhalant (dry powder) or an ophthalmic solution, other than via parenteral injection or transfusion, to a virus target organ. A nitric oxide (NO) inhalation therapy is conducted experimentally as a similar approach; however, NO is toxic at a high concentration and its dosage setting and adjustment are difficult, and NO itself is a mediator of inflammation and oxidative stress, so that contrarily there are concerns that NO might worsen the COVID-19-infected clinical conditions. Particularly, there is a possibility that NO might make a patient with underlying lung health conditions become severely ill.

Glutathione polysulfide has a very significant advantage from the view point of its strong anti-inflammatory and immune control function. In other words, the glutathione polysulfide is also expected to have medical efficacy of suppressing the occurrence of various complications such as the cytokine storm and the pulmonary fibrosis after the infection. Moreover, the point of action is pinpointed at the thiol group which is the activity center of the virus protease, so that an excellent effect of a multidrug therapy treatment with other drugs can be expected. Furthermore, since the GSSSG can be targeted at not only the virus, but also widely at proteases on the host side, it can be also applied as a preventive and therapeutic drug for various viruses including the coronavirus other than the SARS-CoV-2, and the influenza.

Polysulfide as a preferred example of the active sulfur compound has a reduced form and an oxidized form; and R-(S)ₙ-SH (n=1, 2,....) is the reduced form, while R-(S)ₙ-R (n=2,3,....) is the oxidized form.

Fig. 5 shows the results of a SARS-CoV-2 protease inhibition test by the GSSSG. The GSSSG can be produced based on the description of paragraphs [0025] and [0031] of the Japanese Patent Application No. 2017-543669. Recombined papain-like protease (PLpro) and the main protease (3CL^{pro}) which are derived from the SARS-CoV-2 were incubated with GSSSG, Ebselen (2-Phenyl-1,2-benzoisoselenazol-3(2H)-one), and NO (nitric oxide) (respectively 0.3 mM) at 37 degrees Celsius for 15 minutes, then the protease activity was measured by using an MCA fluorogenic substrate and a fluorescence plate reader (excitation wavelength: 380 nm; and fluorescence wavelength: 460 nm).

Referring to Fig. 5, the same level of strong inhibitory activity of the GSSSG on the aforementioned two proteases as that of Ebselen was confirmed as compared to the control (GSSSG(-)). It is reported in NPL 3 and NPL 4 mentioned earlier that Ebselen has strong inhibitory activity on the two proteases. Since the genomes of SARS-CoV-2 are published, PLpro and 3CLpro were synthesized based on such genomes.

Regarding cloning of the papain-like protease and the main protease of SARS-CoV-2, artificial gene synthesis was conducted with respect to papain-like protease (4946-5923) and main protease (10055-10972) regions from the SARS-CoV-2 genomes (accession number: NC_045512). The base sequence was approximated to codon usage of Escherichia coli. Regarding these artificial gene fragments, the papain-like protease was cloned into a pET53 vector and then transformed to NiCo21(DE3), while the main protease was cloned into a pE SUMO vector and then transformed to BL21(DE3). IPTG induction caused the papain-like protease to be expressed as a His-tag fusion protein and caused the main protease to be expressed as a His-tag SUMO-tag fusion protein in Escherichia coli. These recombinant proteins were refined with an Ni-NTA agarose and the main protease recombinant protein further underwent SUMO protease processing, thereby removing His-tags and SUMO-tags.

The papain-like protease and main protease base sequences used for the gene cloning are as follows.

### Papain-like Protease: Sequence Listing 1

### Main Protease: Sequence Listing 2

The concentration of the GSSSG in a formulation is normally 0.0003 µg/mL or more, may be 0.003 µg/mL or more, may be 0.01 µg/mL or more, may be 0.03 µg/mL or more, may be 0.1 µg/mL or more, may be 0.3 µg/mL or more, may be 1 µg/mL or more, may be 3 µg/mL or more, may be 10 µg/mL or more, or may be 30 µg/mL or more. Meanwhile, an upper limit is normally 1000 µg/mL or less, may be 300 µg/mL or less, may be 100 µg/mL or less, or may be 30 µg/mL or less.

Next, models were created by using a SARS-CoV-2 JPN/TY/WK-521 strain (acquired from National Institute of Infectious Diseases) to infect Type II transmembrane serine protease (TMPRSS2) overexpressed African green monkey kidney epithelial cells (VeroE6/TMPRSS2 cells acquired from JCRB Cell Bank) and causing the infected cells to grow (MOI: 0.01), and the anti-SARS-CoV-2 action of the GSSSG was confirmed by means of a quantitative PCR method by using a 2019 novel coronavirus detecting reagent kit (SHIMADZU CORPORATION) and CFX Connect Real-Time System (Bio-Rad Laboratories, Inc.). The models were composed of GSSSG-administered groups (10 µM, 100 µM, and 300 µM) and a no GSSSG-administered group.

Fig. 6 shows the results of an anti-SARS-CoV-2 action test by the GSSSG. Referring to Fig. 6, the test shows the results that an RNA viral load decreases along with an increase in the dosage of the GSSSG.

The glutathione trisulfide can be synthesized based on, for example, WO2018/117186 publication. GS(S)ₙG (n=1-7) can be further synthesized based on pp.7606-7611 /PNAS/ May 27, 2014 / vol. 111 / no. 21 (www.pnas.org/cgi/doi/10.1073/pnas.1321232111). GSH (20 mM) was caused to react with NaHS (20 mM) at room temperature for 15 minutes under the existence of I₂ (20 mM) in a Tris-HCl buffer solution (20 mM) (pH7.4). The reaction mixture was provided for an RP-HPLC in order to refine GS(S)ₙG. The HPLC was conducted by using the Waters e2695 series whose UV detector was set to 210 nm. Samples underwent mobile phases A (H₂O + 0.1% trifluoroacetic acid) and B (methanol) with a linear gradient from 2% to 70% to reach B in 15 minutes at a flow speed of 1 ml/min by using a TSKgel ODS-80Ts column (4.6×150 mm; Tosoh Corporation) at 40 degrees Celsius. Similar effects to those of the GSSSG can be confirmed with respect to each of GS(S)ₙG (n=3-7).

### [Sequence Listing]

B013P20002-PCT(sequence listing)_ST25.txt

## Claims

1. An antiviral drug containing an active sulfur compound as its main medical efficacy component.

2. The antiviral drug according to claim 1, wherein the active sulfur compound is R₁S(S)ₙR₂
(where R₁ and R₂ are selected from amino acids and polypeptides, which independently have a thiol group, and represent parts other than the thiol group; and n is an integer equal to or more than 2).

3. The antiviral drug according to claim 2, wherein the active sulfur compound is formed so that each of the R₁ and R₂ is L-cysteine (Cys), homocysteine (Hcys), or glutathione (GSH).

4. The antiviral drug according to claim 3, wherein the active sulfur compound is glutathione polysulfide.

5. The antiviral drug according to claim 4, wherein the active sulfur compound is GSSSG.

6. The antiviral drug according to any one of claims 1 to 5, wherein the virus is a coronavirus (CoV).

7. The antiviral drug according to claim 6, wherein the coronavirus is a novel coronavirus (SARS-CoV-2).

8. The antiviral drug according to any one of claims 1 to 7, wherein the antiviral drug is a therapeutic drug for viral pneumonia.

9. A preventive and therapeutic drug for a novel coronavirus infection (COVID-19), the drug containing glutathione trisulfide (GSSSG) as its main medical efficacy component.

10. An immune control agent (adjuster) containing glutathione trisulfide (GSSSG) as its main medical efficacy component.

11. An anti-inflammatory drug containing glutathione trisulfide (GSSSG) as its main medical efficacy component.

12. A preventive and therapeutic drug for viral pneumonia, the drug containing an active sulfur compound as its main component.

13. The preventive and therapeutic drug for the viral pneumonia according to claim 12, wherein the active sulfur compound is R₁S(S)ₙR₂
(where R₁ and R₂ are selected from amino acids and polypeptides, which independently have a thiol group, and represent parts other than the thiol group; and n is an integer equal to or more than 2).

14. The preventive and therapeutic drug for the viral pneumonia according to claim 13, wherein the active sulfur compound is formed so that each of the R₁ and R₂ is L-cysteine (Cys), homocysteine (Hcys), or glutathione (GSH).

15. The preventive and therapeutic drug for the viral pneumonia according to claim 14, wherein the active sulfur compound is glutathione polysulfide.

16. The preventive and therapeutic drug for the viral pneumonia according to claim 15, wherein the active sulfur compound is GSSSG.

17. The preventive and therapeutic drug for the viral pneumonia according to claim 12, wherein the drug is a preventive and therapeutic drug for a chronic obstructive pulmonary disease (COPD).
